# EUROPEAN PATENT APPLICATION

(11) **EP 4 835 089 A1**
(43) Date of publication of application: **04.11.2026**
(21) Application number: 25173598.1
(22) Date of filing: 30.04.2025
(51) Int. Cl.: C12P 1/04, A01N 65/00, A23L 35/00, A61K 8/97

(54) **A BACTERIAL EXTRACTION METHOD FOR THE PRODUCTION OF FERMENTED PLANT EXTRACTS AND COMPOUNDS OBTAINED BY THIS METHOD**

(71) Applicant: Aktif Biyokimya AS, 34906 Istanbul (TR)
(72) Inventor: Garipoglu, Can, 34906 stanbul (TR); Yilmaz, Mahmut Selim, 34906 stanbul (TR)
(74) Representative: Bakirci, Utkan Bahri

(57) **Abstract**

The present invention relates to a method for fermenting plant material using enzyme-producing bacteria and extracting bioactive compounds through fermentation. This method enables the plant cell structures to be broken down naturally by microorganisms, thereby allowing the bioactive compounds to be obtained with higher yield and enhanced bioavailability. The invention provides a sustainable and environmentally friendly extraction method without the use of chemical solvents. It enables the production of fermented plant extracts that can be used in the cosmetics, food, pharmaceutical, and agricultural industries.

## Description

### Technical Field of the Invention

The present invention relates to a method for fermenting plant material using enzyme-producing bacteria and extracting bioactive compounds through fermentation, to the compounds obtained by this method, and to mixtures containing these compounds.

### State of the Art

The process of obtaining plant extracts is a critical method involving the removal of biologically active components from plant material. These extracts contain bioactive compounds naturally found in plants, such as essential oils, polyphenols, alkaloids, and flavonoids, and are widely used in the medical, cosmetic, food, and pharmaceutical industries.

In the current state of the art, various methods are available for plant extraction, and each method has different advantages and disadvantages in terms of efficiency, the stability of the obtained bioactive compounds, environmental impact, and the bioavailability of the extract.

Current techniques aim to release the bioactive compounds contained in plant cells by breaking down the plant cell structures through physical, chemical, or thermal means. The commonly used techniques today include cold pressing, steam distillation, supercritical carbon dioxide extraction, solvent extraction, ultrasonic extraction, and microwave extraction.

In the cold pressing method, plant material is mechanically compressed at low temperature to obtain essential oils and other bioactive components. It is especially preferred for oil extraction from citrus peels and certain seeds. However, the biggest disadvantage of this method is the low yield due to the absence of heat.

In the steam distillation method, the plant material is processed with steam to separate the volatile components. Then, through a cooling process, the essential oils and other compounds are collected in liquid phase. It is widely used for essential oil production. However, there is a risk of degradation of heat-sensitive compounds due to the effect of steam.

In traditional solvent extraction, various organic solvents (such as ethanol, methanol, hexane) are used to dissolve plant material and perform the extraction. The solvent is then evaporated to obtain a concentrated extract. The use of chemical solvents (e.g., hexane, ethanol) may, in some cases, leave undesirable residues in the final product. Processes that require high temperatures may cause the degradation of certain active compounds. Moreover, due to environmental impacts and toxicity risks, the sustainability of this method is questionable.

In the supercritical carbon dioxide (CO₂) extraction method, CO₂ is brought to a supercritical state under high pressure and specific temperature conditions, making it act as a solvent for plant compounds. This extraction method must operate effectively under specific temperature and pressure conditions at high pressure, which increases the processing cost. Additionally, it may not be the most effective method for the extraction of certain compounds, such as polyphenolic compounds.

Among the current techniques, modern extraction techniques such as ultrasonic and microwave-assisted extraction also have certain disadvantages.

In the ultrasonic extraction method, ultrasonic waves are used to break down plant cell walls and release bioactive compounds. However, this method can be costly due to high energy consumption, and in some cases, prolonged processing time may reduce production efficiency. Furthermore, since ultrasonic waves must be applied at optimal frequencies and durations, using incorrect parameters may result in low extraction efficiency.

In the microwave-assisted extraction method, microwave energy is used to heat water molecules within the plant cells, thereby breaking the cell wall and releasing bioactive compounds. However, this method may increase costs due to the requirement for specialized equipment, and microwave energy may alter or degrade the structure of certain compounds. Additionally, since microwave energy needs to be applied in an intense and targeted manner, improper parameter settings may lead to undesirable molecular changes.

Although traditional and modern extraction methods each offer specific advantages for different plant components, they also have certain significant limitations. The use of chemical solvents may carry the risk of residue and may degrade some bioactive compounds, while high-temperature processes may lead to the degradation of heat-sensitive components. Moreover, methods such as supercritical CO₂, ultrasonic, and microwave-assisted extraction require specialized equipment, making them costly. In addition, most methods do not fully enable the selective extraction of the targeted bioactive compounds, which may reduce the efficiency and effectiveness of the extract.

In addition, enzyme-assisted extraction is being used as a more recent technique. In this technique, the cellular structure of the plant material is broken down with the help of enzymes, enabling the bioactive compounds to be released more effectively.

Enzymes are proteins that facilitate biological processes by converting large molecules into smaller or structurally different molecules. Enzymes are also proteins that accelerate chemical reactions in biological systems. Without them, cellular processes would occur very slowly or not at all. Enzymes are usually specialized for a specific type of reaction and bind to specific substrates to either break them down into smaller molecules (catabolism) or form new compounds (anabolism). Therefore, enzymes are biocatalysts that accelerate chemical reactions in biological systems, and since they remain unchanged at the end of the reaction, they can be reused multiple times. Enzymes that act specifically on a particular substrate exhibit high specificity and function most efficiently within an optimal temperature and pH range. Their mechanism of action consists of three main steps: First, the enzyme binds to a specific substrate to form an enzyme-substrate complex. Then, it weakens the chemical bonds of the substrate or forms new bonds to carry out the reaction. In the final step, the products are released and the enzyme becomes ready for the next reaction. This process enables metabolic reactions in living organisms to proceed rapidly and in an orderly manner.

Enzymes that break down plant cell walls have important applications especially in the fields of food, pharmaceuticals, and biotechnology. Plant cell walls are composed of complex polysaccharides such as cellulose, hemicellulose, pectin, and lignin. These structures can make it difficult for the bioactive compounds within the cells (such as phenolic compounds, flavonoids, antioxidants, vitamins, and essential oils) to be released. Enzymes that degrade plant cell walls enable the bioactive compounds inside the cells to be released with higher efficiency. For example, hydrolytic enzymes break down the cell wall, thereby allowing these compounds to be extracted with higher yield.

Through the use of these enzymes, the yield of bioactive compounds obtained from plant sources can be increased, resulting in more effective and natural products. The main types of enzymes that can be used in enzyme-assisted extraction technologies can be exemplified according to their working mechanisms as follows:
- Cellulases → Weaken the cell wall by breaking down cellulose.
- Pectinases → Break down pectin to dissolve intercellular connections.
- Hemicellulases → Break down hemicelluloses to make the cell wall more permeable.
- Laccase and Peroxidases → Modify the lignin structure to aid in extraction.

The use of enzymes in this way is directly related to natural biological processes. Plants actively utilize enzymes in their own mechanisms of growth, defense, and recycling. Enzymes that degrade cell wall components are naturally present in plants and perform various biological functions.

Growth and Development: As plants grow, they produce enzymes such as cellulase, pectinase, and hemicellulase to expand and reshape their cell walls. These enzymes help the cells expand and support plant growth by breaking down the polysaccharides in the cell wall in a controlled manner.

Fruit Ripening: During the fruit ripening process, pectinase and cellulase enzymes break down the pectin in the cell wall, leading to the softening of the fruit. This process is a significant biochemical event that naturally alters the taste and texture of the fruit.

Defense Mechanisms: Plants produce enzymes to protect themselves against pathogens. For example, glucanases and chitinases break down the cell walls of fungi that infect the plant, thereby inhibiting the growth of the pathogens.

Programmed Cell Death and Leaf Abscission: Under aging or stress conditions, plants use hydrolytic enzymes to eliminate specific cells. During leaf abscission, enzymes such as pectinase and cellulase break down the cells in the petiole, making it easier for the leaf to detach.

Seed Germination: During germination, enzymes that break down cell wall components are used to allow the embryo to grow. For example, β-glucanases and hemicellulases break down complex carbohydrates in the cell wall, enabling them to be used as an energy source.

The enzymatic degradation that naturally occurs in plants forms the basis of processes used in biotechnology to release bioactive compounds. For example, pectinase enzymes, which mimic the naturally occurring enzymes during fruit ripening, can be used in fruit juice production. Therefore, plants already utilize enzymes in their natural processes to regulate cell wall degradation. Industrial processes benefit from enzymes by mimicking these natural biochemical pathways to extract or process plant components more efficiently.

Considering the structure of plant cells, certain enzymes play a critical role in breaking down from the outside of the cell inward to release active compounds. For plants with high cellulose content, cellulase enzyme is used; for those with high pectin content, pectinase enzyme; for breaking down the cell membrane, protease enzymes; and for breaking down tannins stored in plant cells, tannase enzyme is used. For instance, the tannase enzyme breaks down gallotannins in plants with high tannin content, such as the rose plant, to produce gallic acid, which increases the antioxidant level of the extract.

Current enzyme-assisted extraction methods are generally applied by directly adding purified enzymes. While this increases production costs, the effectiveness and stability of the enzyme may also decrease over time. In addition, if the use of purified enzymes is not optimized according to specific plant species and cellular structures, the extraction yield may be low.

Another issue is the difficulty in determining enzyme profiles suitable for the cellular structure of the plant material. In the methods available in the art, certain predefined enzymes are added to the plant material; however, since each plant has a different cellular structure, a fixed enzyme profile may not be suitable for every material. Furthermore, fermentation-based extraction processes are generally not standardized and are not optimized for the efficient extraction of specific bioactive compounds using specific microorganisms.

Examples of the methods found in the current art include the patent documents US2005089499A1 and WO2014148711A1.

The patent document US2005089499A1 describes a method in which a fermentation medium containing plant components or extracts is fermented with microorganisms to produce cosmetic and/or pharmaceutical active ingredients. In addition, cosmetic or pharmaceutical products used for skin and hair care that contain these active ingredients, and methods of their use, are also described. However, in the method disclosed in this patent, the use of chemicals such as alcohol to obtain plant extracts may lead to the loss of certain natural compounds during the process and may render the subsequent fermentation production process complex and costly. Moreover, the separation of the obtained active ingredients into molecular fractions using chromatographic methods may require additional purification steps, making the production process more laborious and costly.

The patent document WO2014148711A1 describes a cosmetic composition containing plant extracts or their fermentation products, and a method for producing the same. This composition contains an extract obtained from one or more plants such as Taraxacum platycarpum, Centella asiatica, Ecklonia stolonifera, Bambuseae, Petasites japonicus, Chamaecyparis obtusa, Cactaceae, and Aloe by means of hot water extraction, ethanol extraction, microwave extraction, or supercritical extraction, or a component fermented with Lactobacillus. This composition can be used as an active ingredient in functional cosmetic products. However, since known extraction techniques are used in this method, certain technical disadvantages still remain. Primarily, it is possible for some bioactive compounds to be lost or chemically altered during the extraction process. In addition, mixing a plant extract obtained through conventional extraction techniques with a subsequently fermented component increases the complexity of the process and may hinder the efficient recovery of bioactive compounds compared to performing fermentation directly on the plant material.

As a result, in order to overcome the above-mentioned shortcomings, there is a need for a more economical and sustainable enzymatic extraction method that eliminates the use of chemical solvents. Furthermore, there is a need for a process that performs extraction using enzymes naturally produced by microorganisms, with enzyme production optimized specifically for the plant material.

### Detailed Description of the Invention

The invention relates to a bacterial extraction method for the production of fermented plant extracts. This method may be referred to as Bacterial Extraction Targeting Actives (BETA) technology.

Bacterial fermentation is the process by which microorganisms (particularly bacteria) chemically break down organic matter, with or without the presence of oxygen, to produce energy and generate various metabolites. This process is used in many fields, from food production to the pharmaceutical industry. During fermentation, bacteria typically metabolize carbohydrates (such as sugars) to produce acids, alcohols, gases, and other byproducts. Bacterial fermentation is a process commonly observed in nature and naturally occurs in soil, in the digestive systems of animals, in aquatic environments, and during the decomposition of plant materials. The industrial application of fermentation in accordance with the method of the invention has been developed by optimizing these natural processes.

One aim of the invention is to enable the enzymatic extraction of bioactive compounds from plant materials using bacterial fermentation. In this process, enzymes produced by natural or probiotic microorganisms break down the plant cellular structure, thereby releasing bioactive compounds and enhancing their bioavailability.

In the method related to the invention, bacteria are used as powerful biofactories for industrial enzyme production. The enzymes produced in this way are already widely used in the fields of food, pharmaceuticals, biotechnology, detergents, agriculture, and environmental applications, accelerating production processes and reducing costs. Today, thanks to synthetic biology and genetic engineering, it has become possible to produce more efficient and customized enzymes from bacteria.

Another aim of the invention is to ferment plant material using enzyme-producing bacteria and, through this natural biological process, to break down plant cell structures in order to obtain bioactive compounds with high yield and enhanced bioavailability, without the adverse effects of chemical solvents and high processing temperatures.

Furthermore, the invention aims to develop targeted extraction methods using bacterial enzymes. The adaptation of bacteria to the plant matrix to be extracted and the optimization of the extraction process are intended to ensure a high-quality and controlled fermentation process.

The BETA technology subject to the invention offers an environmentally friendly extraction method by using microbial enzymes instead of chemical solvents and enables bioactive compounds to become more stable and have higher bioavailability.

The invention, in its most general form, relates to a bacterial extraction method for the production of fermented plant extracts. This method comprises the following steps:
a) selecting at least one plant material and determining the enzyme requirement necessary for the extraction of bioactive compounds, and selecting suitable types of microorganisms for the production of these enzymes;
b) preparing a culture medium (growth medium) containing at least one carbon source, at least one protein source, and preferably additional nutrients;
c) adapting (exposing) at least one selected microorganism to increasing concentrations of the plant extract over multiple fermentation cycles;
d) after the adaptation is complete, preparing the optimal culture medium in which fermentation will take place and adding the plant extract to this medium;
e) sterilizing the prepared mixture in a way that preserves the active ingredients of the plant components, then cooling it to the determined optimal temperature, and initiating the fermentation process by adding a bacterial culture prepared under sterile conditions;
f) subjecting the fermented culture medium to heating and/or filtration in order to obtain the fermented plant extract containing bioactive compounds.

In this method, at least one plant material is used, and bioactive compounds are obtained from this material.

In one embodiment of the invention, the plant to be fermented may be selected from one or more of the following sources: fruit and vegetable sources rich in polyphenols and flavonoids such as grape skin, green tea, whole pomegranate, blueberry, strawberry, raspberry, blackberry, apple, and artichoke; medicinal and aromatic plants known for their antioxidant and antibacterial properties such as turmeric, ginger, aloe vera, basil, thyme, sage, rosemary, mint, chamomile, lavender, rose, lemongrass, and echinacea; cereals and legumes rich in fiber and protein such as oats, wheat bran, soybeans, chickpeas, and lentils; and plant extract sources with high functional compound content such as citrus pulp, grape seed extract, and cocoa husk.

The determination of the enzymatic profile, as defined in step (a) of the method subject to the invention, refers to identifying which enzymes are required to ensure that the enzymes produced by bacteria effectively break down the plant material and release the bioactive compounds. Since the aim of the invention is to perform fermentation using enzyme-producing bacteria, this step is critical for identifying bacteria with high enzyme production capacity and understanding which enzymes will be more effective in the plant extraction process. In this process, since each plant material contains different cellular components, different enzymes will be needed to break them down. More specifically, cellulase enzyme is required for plants with high cellulose content such as wheat bran, rice husk, corn stalk, bamboo, cotton fiber, and sugarcane bagasse; pectinase enzyme is required for plants with high pectin content such as apple pomace, citrus peels (e.g., orange, lemon, mandarin), stone fruits like peach, apricot, and plum, tomato pomace, and carrot pulp; protease enzyme is needed for protein-rich plants such as soybeans, peas, lentils, chickpeas, hazelnuts, walnuts, and almonds; and tannase enzyme is required for tannin-rich plants such as green/black tea leaves, cocoa husk, grape skin and seeds, oak bark, and pomegranate peel. For example, if a plant with high cellulose and pectin content such as grape skin or citrus pulp is selected for fermentation, bacteria that produce cellulase and pectinase enzymes would be the most suitable option. In such a case, wild-type bacterial strains with high enzyme production capacity may be identified.

In one embodiment of the invention, the selected bacterium is a probiotic such as Lactobacillus, Saccharomyces, Bacillus subtilis, Pediococcus spp., Leuconostoc spp., Enterococcus, or Streptococcus thermophilus. Here, Lactobacillus refers to a genus that includes many different species, such as Lactobacillus plantarum and Lactobacillus rhamnosus. Therefore, the term Lactobacillus generally refers to the entire genus, while specific species within this genus may be used depending on the application.

Pediococcus spp. are lactic acid bacteria used in fermentation processes and support the production of bioactive compounds.

Leuconostoc spp. possess probiotic properties, and species that synthesize polysaccharides are particularly used in fermentation processes.

Enterococcus spp. are naturally present in fermentation processes and may contribute to the stabilization of bioactive compounds.

Streptococcus thermophilus is a probiotic bacterium commonly used in food fermentation.

In one embodiment of the invention, fermentation and extraction processes similar to those described above can also be carried out using microorganism species, yeasts, and/or fungi that are not classified as probiotics. These may be used alone or as a mixture composed of at least two selected species. In one embodiment, yeast species such as Kluyveromyces marxianus, Pichia pastoris, or Candida utilis, and Yarrowia lipolytica may be used. In another embodiment, fungal species such as Aspergillus oryzae, Aspergillus niger, Trichoderma reesei, Rhizopus oryzae, Pleurotus ostreatus, or Saccharomyces cerevisiae may be used.

Examples of non-probiotic bacteria include Acetobacter, Komagataeibacter, and Pseudomonas putida. Pseudomonas putida is an important bacterium in biotechnology in terms of enzyme production and metabolic activity. Acetobacter spp. are particularly suitable for the production of organic acids and the extraction of bioactive compounds. Komagataeibacter spp. are used in cellulose production and organic acid fermentations.

In other words, in the fermentation process, at least one species is used from among the following: probiotic bacteria such as Lactobacillus, Streptococcus thermophilus, Pediococcus, Leuconostoc, or Enterococcus; non-probiotic bacteria such as Pseudomonas putida, Acetobacter, Komagataeibacter, Bacillus subtilis, or Clostridium butyricum; yeasts such as Saccharomyces, Kluyveromyces marxianus, Pichia pastoris, Candida utilis, or Yarrowia lipolytica; and fungi such as Aspergillus oryzae, Aspergillus niger, Trichoderma reesei, Rhizopus oryzae, Saccharomyces cerevisiae, or Pleurotus ostreatus.

In one embodiment of the invention, species from the Lactobacillus genus such as Lactobacillus plantarum or Lactobacillus rhamnosus may be used.

In different embodiments of the invention, the following mixtures are used:

| | |
|---|---|
| 1. | Mixture: Lactobacillus plantarum and Lactobacillus rhamnosus |
| 2. | Mixture: Lactobacillus plantarum and Saccharomyces cerevisiae |
| 3. | Mixture: Lactobacillus rhamnosus and Saccharomyces cerevisiae |
| 4. | Mixture: Bacillus subtilis and Lactobacillus rhamnosus |
| 5. | Mixture: Bacillus subtilis and Saccharomyces cerevisiae |

Briefly, in step (a), the type of plant material to be used is determined, and then, based on the structure of this material, the necessary enzymes are identified. At this stage, the bacteria that may be used depending on the required enzyme are selected; however, in the subsequent stage, it is necessary to measure the actual enzyme production capacity in order to identify the most effective bacterium. This is carried out during the adaptation process.

In steps (b) and (c), the actual enzyme production capacity is measured. First, in step (b), a culture medium is prepared by selecting appropriate carbon, protein, and additional nutrient sources in order to create a suitable growth environment for the bacterium and to support enzyme production. In one embodiment of the invention, the carbon source is molasses, potato starch, or corn starch, and the nitrogen source is hydrolyzed protein, hydrolyzed vegetable protein, yeast extract, or peptone. In one embodiment of the invention, a culture medium containing 3% molasses, 2% hydrolyzed vegetable protein, and 1% oats has been prepared.

In step (c), the selected at least one microorganism is gradually adapted to the plant extract, and bacterial compatibility is tested and optimized. By observing the bacterial strains that are gradually adapted to the plant extract, the strains that exhibit the best compatibility with the plant components can be identified. In order to enhance the metabolic activity of the bacterium and optimize enzyme production, it is important to gradually increase the concentration of the plant extract. In one embodiment of the invention, during the adaptation process, 1% plant extract is first added to the defined culture medium, followed by 3% plant extract.

More specifically, the adaptation period consists of two stages: in the first stage, the microorganism is exposed to a culture medium containing a low concentration of plant extract in order to initiate its metabolic response and stimulate enzyme production, and fermentation is carried out under predetermined temperature and agitation conditions. In the second stage, the microorganism is transferred to a culture medium containing an increased concentration of plant extract in order to enhance its tolerance to the extract and improve enzymatic activity, and fermentation is again carried out under predetermined temperature and agitation conditions. This process is repeated iteratively over multiple fermentation cycles, with the concentration of the plant extract being gradually increased at each stage.

The temperature and agitation rate used during the adaptation process are determined based on the physiological characteristics and optimal growth conditions of the selected microorganism. Each microorganism species exhibits its highest metabolic activity within a specific temperature range and at specific agitation speeds that maintain the oxygen/carbon dioxide balance. For example, Lactobacillus species typically show optimal growth around 37°C, while yeast species such as Saccharomyces cerevisiae perform more efficiently at temperatures of 28-30°C. Similarly, the agitation speed (rpm) is adjusted according to the microorganism's oxygen requirements and cell structure; lower speeds are suitable for anaerobic bacteria, whereas aerobic or yeast species perform better at higher agitation speeds. Therefore, the adaptation process is structured according to the specific biological requirements of each microorganism, ensuring that both enzyme production and adaptation to bioactive plant compounds are achieved in the most efficient manner.

For example, in the case of using Lactobacillus plantarum during the adaptation process, the culture medium is prepared to contain 1% plant extract, and incubation is carried out at 37°C and 120 rpm agitation for 24 hours. These conditions promote enzyme production by L. plantarum while preserving cell integrity and enabling gradual adaptation to the bioactive compounds in the extract.

In one embodiment of the invention, during the adaptation process defined in step (c), the concentration of plant extract added to the culture medium is gradually increased; in other words, the concentration of plant extract in the culture medium used in the second stage is higher than that in the first stage. Therefore, the first stage is referred to as involving a 'low concentration', while the second stage is referred to as involving an 'increased concentration'.

In one embodiment of the invention, the low concentration ranges between 1% and 3%. In another embodiment of the invention, the increased concentration ranges between 3% and 10%. In other words, in one embodiment of the invention, the culture medium contains 1-3% low-concentration plant extract in the first stage and/or 3-10% increased-concentration plant extract in the second stage.

In one embodiment of the invention, step (c) of the method, in which the microorganism is gradually adapted to the plant extract, comprises exposing the microorganism to a culture medium containing a low concentration of plant extract in the first stage, followed by fermentation under predetermined temperature and agitation conditions; and in the second stage, transferring the microorganism to a culture medium containing an increased concentration of plant extract, followed by fermentation under the same predetermined temperature and agitation conditions. These two stages may not be sufficient for complete adaptation. In such cases, bacterial colonies are selected again, and the process is continued iteratively. In other words, if the adaptation process is not completed in two stages, the first two stages are repeated over multiple fermentation cycles, and the concentration of the plant extract is gradually increased at each stage.

In one embodiment of the invention, the defined optimal culture medium contains 5-10% molasses, 2-5% hydrolyzed protein, and 2-5% oats. To this medium, 10% to 40% plant extract is added in step (d).

In another embodiment of the invention, the defined optimal culture medium contains 5% molasses, 2% hydrolyzed vegetable protein, and 2% oats. To this medium, 10% to 40% plant extract is added in step (d).

In a further embodiment of the invention, in addition to the contents of the optimal culture medium, cereal flours are dry-mixed at 30-40% wheat flour, 20-30% barley flour, 20-30% corn flour, and 10-20% rye flour. The resulting mixture is used at a level of 2-5% to provide a sufficient nutrient medium for bacterial growth.

In one embodiment of the invention, the method of using the plant material is as follows: The plant is washed with water, then broken into smaller pieces to increase surface area. The resulting material-including the fruit's seeds, peel, and flesh without separation-is directly added to the culture medium. Dried plants are ground into powder using a micronizing grinder and directly added to the culture medium in powdered form. The raw materials are used directly in fermentation.

In step (e), the plant extract mixture added to the optimal culture medium is subjected to a sterilization process in a manner that preserves the active ingredients of the plant components. In one embodiment of the invention, this sterilization process is carried out at 55°C for 60 minutes, ensuring that the active compounds are preserved. After sterilization, the mixture is cooled to a predetermined optimal temperature. The mentioned optimal temperature refers to the temperature at which the culture can grow, which may range between 20-40°C. Once the optimal temperature is reached, a sterile-prepared bacterial culture is added to the fermentation medium at a ratio of 3%. In one embodiment of the invention, the fermentation process lasts for 24 to 48 hours.

In step (f), following fermentation, the culture medium is subjected to heating and filtration processes. The filtration process is initially performed by passing the mixture through sieves with a mesh size of 250 microns to remove large plant or fruit particles. Subsequently, the product is passed through an 80-micron stainless steel filter with the help of a pump to remove undesirable particles. Instead of using fine filters to clarify the products, 0.1-0.5 grams of gelatin dissolved in 5-10 grams of hot water per 1 kg of product is added to the mixture, and the product is allowed to rest in a container for three days. During this resting period, suspended plant or fruit particles settle at the bottom. The upper layer is then collected to obtain a product clear enough to pass through a 10-micron filter. In one embodiment of the invention, in step (f), the mixture is heated at 70°C for 45 minutes.

In every application, a heating step is applied. The reason for this is that heating damages the cell membrane and cell wall of the cultured bacteria, thereby inhibiting their activity and allowing beneficial molecules (postbiotics) contained within the bacterial cell membrane to be released into the product medium. In this way, an extract suitable for the skin microbiota is obtained. The benefits of postbiotics for the skin microbiota have been mentioned in numerous publications. Therefore, instead of removing the bacteria by filtration or high-speed centrifugation, the release of their intracellular content is ensured.

The determination and optimization of fermentation parameters is a critical step in the development of the invention, which is a biotechnological process. Various experimental studies have been conducted to establish the optimal growth conditions for the bacteria, and systematic approaches and nonlinear dynamic analyses have been used to ensure that the fermentation process proceeds in the most efficient manner.

The fermentation medium has been designed based on two main variables: the plant material to be fermented and the bacterial strain to be used. The plant material is selected according to the desired bioactive compounds to be obtained, while bacterial strains are determined based on their enzyme production capacity. In addition, in order to support bacterial growth and enzyme production, the optimal ratios of carbohydrate and protein sources have been established through experimental studies, and the fermentation medium has been optimized in accordance with these parameters.

In one embodiment of the invention, gallic acid and/or pyrogallol compounds are obtained through the enzymatic processing of plant materials containing gallotannins. The tannase enzyme produced by certain microorganisms such as Lactobacillus enables important biotransformations by hydrolyzing the ester bonds present in the structure of gallotannins. In this embodiment, plant sources rich in gallotannins, such as rose or lavender, are used, and the tannase enzyme is applied to these materials to hydrolyze the ester bonds in the gallotannin structure. As a result of this reaction, galloyl groups are released to form gallic acid. Subsequently, decarboxylation of gallic acid is carried out by the action of the enzyme gallic acid decarboxylase, thereby producing pyrogallol. As a result of these successive enzymatic conversions, the obtained plant extract offers a composition rich in gallic acid and pyrogallol, particularly notable for its high antioxidant activity.

In addition, another embodiment of the invention comprises the production of extracts rich in ellagic acid and/or urolithin A through the enzymatic hydrolysis and fermentation of plant materials containing ellagitannins. Ellagitannins can be processed through similar hydrolysis mechanisms to allow for the production of ellagic acid. In this context, plant sources rich in ellagitannins, such as pomegranate, are used, and tannase or similar hydrolytic enzymes are applied to the raw materials. This enables the cleavage of bonds in the ellagitannin structure, resulting in the release of hexahydroxydiphenic acid (HHDP). Subsequently, under appropriate low pH conditions or due to specific environmental factors, HHDP undergoes a lactonization reaction to form the ellagic acid compound. The ellagic acid obtained is subjected to decarboxylation, dehydroxylation, and other structural modifications during the fermentation process carried out with selected microorganisms, sequentially forming intermediate metabolites such as urolithin M5, D, and C. Through the successive conversion of these metabolites, urolithin A-a biologically active and stable form-is ultimately produced. The extract prepared in this embodiment provides a functional composition rich in ellagic acid and urolithin A, offering particularly antioxidant, anti-inflammatory, and cell-regenerating properties.

In addition, another embodiment of the invention comprises the production of modified flavonoid compounds through the fermentation of plant materials containing flavonoids using Lactobacillus species and similar microorganisms. During fermentation processes, enzymes produced by Lactobacillus and related microorganisms are capable of modifying not only tannins but also other phenolic compounds such as flavonoids. In this embodiment, glycosidase enzymes such as β-glucosidase, rhamnosidase, and similar enzymes secreted by these microorganisms hydrolyze the glycosidic bonds present in the structure of flavonoid molecules, thereby enabling their transformation into different chemical forms. For example, at the end of the fermentation process, modified compounds such as kaempferol 3-O-rhamnoside can be obtained. These compounds offer advantages such as higher bioavailability, enhanced antioxidant activity, and improved intestinal absorption compared to the original flavonoids. In this way, the plant extract containing fermentation-modified flavonoids forms a composition that is functionally enriched and biologically more active.

In one embodiment of the invention, combined extraction techniques may be applied. In this case, not only fermentation but also at least one traditional or modern extraction method is included to establish an optimized process. In this embodiment, in addition to the fermentation process, at least one of the following techniques is applied: cold pressing, steam distillation, supercritical carbon dioxide extraction, solvent extraction, ultrasonic extraction, and/or microwave extraction.

The present invention offers a natural and organic extraction method that does not require chemical solvents, thereby increasing the naturalness and reliability of the resulting plant extracts. Since this method enables extraction based entirely on biological processes without leaving chemical residues, it provides a more sustainable and environmentally friendly alternative for both consumers and producers. Moreover, thanks to the fermentation method, all components of the plant material are extracted in a more holistic manner, allowing the full potential of the plant constituents to be realized.

In one embodiment of the invention, nanotechnology is utilized to enhance extraction efficiency and improve the stability of bioactive compounds. In this embodiment, nanoparticles are used and/or nanoencapsulation techniques are applied.

Different bioreactor designs and configurations may be developed to optimize or modify the fermentation conditions related to the invention.

The present invention enhances the concentration of certain bioactive compounds, resulting in extracts that are more potent and effective. The natural fermentation process can also increase the stability of some compounds, thereby extending the shelf life of the products and helping to preserve their efficacy. In addition, this process enables the transformation of certain industrial waste materials, contributing to a more sustainable production process.

The present invention, which has a wide range of applications, allows for the use of the resulting fermented extracts in various sectors such as cosmetics, food, agriculture, and environmental technologies. Furthermore, its ability to adapt to different plant species and compounds facilitates the development of products suitable for various market segments and consumer needs.

In addition, the plant extraction process carried out using the improved natural fermentation method of the present invention offers various advantages identified through technical and academic analyses. These advantages are detailed below:
Biological Activity Profile: The fermented extracts obtained by the present invention can enhance or modify the biological activity of compounds under different fermentation conditions. This may enable the discovery of new bioactive compounds that could be effective in the treatment or prevention of certain diseases.

Component Interactions: The fermentation process can alter the interactions among plant constituents (phytochemicals, vitamins, minerals) and generate synergistic effects. This synergy may enhance the efficacy of bioactive compounds and improve the functional properties of the resulting products.

Microbial Synergy: Specific bacterial strains used in the current method may interact with the microbial flora present on the plant material, giving rise to unexpected biological benefits or new functional properties.

Gene Expression Modification: The fermentation process may modify gene expression within plant cells, thereby increasing the production of secondary metabolites. This can result in significant changes in the synthesis of biologically active compounds and improve the production efficiency of bioactive substances.

Long-Term Stability and Storage: Fermented products possess long-term stability and can retain their biological activity during storage. This method extends shelf life, reduces costs, and enables the production of higher-quality and longer-lasting products for consumers.

Economic and Social Impact: The present invention supports sustainable production processes by promoting the utilization of local agricultural resources and contributing to economic development. Fermentation of locally sourced plant materials may positively impact the agricultural sector and regional development by generating employment and economic value.

Through the method subject to the invention, bioactive fermented extracts can be produced for skin care products in the cosmetics industry and used as raw materials in such products. In the food industry, fermented plant compounds may be obtained for use in functional foods. In the pharmaceutical sector, the fermented plant extracts can be used not only in the production of herbal medicines but also as raw materials in the formulation of probiotic and postbiotic supplements. In the agricultural sector, bioactive fermented extracts may be developed to promote plant growth or increase resistance to disease. In addition, fermented plant extracts may be utilized in the production of biological protectants for natural pest control.

In summary, the invention relates to a method for obtaining bioactive compounds from plant material, comprising the steps of: selecting at least one plant material and determining a suitable enzymatic profile for extracting bioactive compounds; preparing a culture medium containing at least one carbon source, at least one protein source, and optionally additional nutrients; gradually adapting (exposing) at least one selected microorganism to the plant extract, by subjecting it to increasing concentrations of the plant extract over multiple fermentation cycles; once adaptation is complete, preparing the optimal culture medium for fermentation and adding the plant extract to this medium; sterilizing the prepared mixture in a manner that preserves the active components of the plant material, then cooling it to the predetermined optimal temperature, adding the sterile bacterial culture under sterile conditions to initiate the fermentation process; and subjecting the fermented culture medium to heating and/or filtration to obtain a fermented plant extract containing bioactive compounds.

The method of the invention has many areas of application, including food, biotechnology, pharmaceuticals, and cosmetics; the method is also applicable in biofuel production and environmental applications.

Furthermore, the compounds obtained through the method of the invention, as well as mixtures containing these compounds that are suitable for various applications, are also within the scope of protection. More specifically, the invention also covers a cosmetic composition, a food composition, a biostimulant composition, or a bioremediation composition comprising at least one fermented plant extract obtained by the extraction method described in the present invention.

## Claims

1. A method for the extraction of bioactive compounds from at least one plant material, **characterized by** comprising the steps of:
a) selecting at least one plant material and determining a suitable enzymatic profile for the extraction of bioactive compounds;
b) preparing a culture medium (growth medium) comprising at least one carbon source, at least one protein source, and optionally additional nutrients;
c) adapting at least one selected microorganism to increasing concentrations of the plant extract over multiple fermentation cycles;
d) after completion of the adaptation, preparing the optimal culture medium for fermentation and adding the plant extract to said medium;
e) sterilizing the prepared mixture in a manner that preserves the active compounds of the plant components, subsequently cooling it to a predetermined optimal temperature, and initiating the fermentation process by adding a bacterial culture prepared under sterile conditions;
f) subjecting the fermented culture medium to a heating and/or filtration process to obtain a fermented plant extract containing bioactive compounds.

2. The method according to claim 1, wherein the plant material is selected from one or more of the following plant extract sources: fruit and vegetable sources rich in polyphenols and flavonoids such as grape skin, green tea, whole pomegranate, blueberry, strawberry, raspberry, blackberry, apple, and artichoke; medicinal and aromatic plants known for their antioxidant and antibacterial properties such as turmeric, ginger, aloe vera, basil, thyme, sage, rosemary, mint, chamomile, lavender, rose, lemongrass, and echinacea; cereals and legumes rich in fiber and protein such as oats, wheat bran, soybeans, chickpeas, and lentils; and plant extract sources with high functional compound content such as citrus pulp, grape seed extract, and cocoa husk.

3. The method according to claim 1 or 2, wherein at least one microorganism selected from the following is used in the fermentation process: probiotic bacteria such as Lactobacillus, Streptococcus thermophilus, Pediococcus, Leuconostoc, or Enterococcus; non-probiotic bacteria such as Pseudomonas putida, Acetobacter, Komagataeibacter, Bacillus subtilis, or Clostridium butyricum; yeasts such as Saccharomyces, Kluyveromyces marxianus, Pichia pastoris, Candida utilis, or Yarrowia lipolytica; and fungi such as Aspergillus oryzae, Aspergillus niger, Trichoderma reesei, Rhizopus oryzae, Saccharomyces cerevisiae, or Pleurotus ostreatus.

4. The method according to any one of the preceding claims, wherein the carbon source is molasses, potato starch, or corn starch, and the nitrogen source is hydrolyzed protein, hydrolyzed vegetable protein, yeast extract, or peptone.

5. The method according to claim 4, wherein, in step (b), the carbon source is molasses, the protein source is hydrolyzed vegetable protein, and the additional nutrient is oats.

6. The method according to claim 5, wherein the culture medium in step (b) comprises 3% molasses, 2% hydrolyzed vegetable protein, and 1% oats.

7. The method according to any one of the preceding claims, wherein, in step (c), it comprises the steps of:
- in a first stage, exposing the microorganism to a culture medium containing a low concentration of plant extract in order to initiate its metabolic response and stimulate enzyme production, and performing fermentation under predetermined temperature and agitation conditions;
- in a second stage, transferring the microorganism to a culture medium containing an increased concentration of plant extract in order to enhance its tolerance to the extract and improve its enzymatic activity, and performing fermentation under predetermined temperature and agitation conditions;
- and, in the event that the adaptation process is not completed in two stages, repeating the first two stages over multiple fermentation cycles, with a gradual increase in the concentration of plant extract at each stage.

8. The method according to claim 7, wherein the fermentation is carried out for a duration of 48 hours.

9. The method according to claim 7 or 8, wherein in the first stage, the culture medium contains 1% to 3% plant extract at a low concentration and/or in the second stage, the culture medium contains 3% to 10% plant extract at an increased concentration.

10. The method according to any one of the preceding claims, wherein the optimal culture medium, in step (d), comprises 5% to 10% molasses, 2% to 5% hydrolyzed protein, and 2% to 5% oats.

11. The method according to any one of the preceding claims, wherein in step (e), 10% to 40% plant extract is added to the optimal culture medium.

12. The method according to any one of the preceding claims, wherein in step (e), sterilization is carried out at 55°C for 60 minutes and/or, after reaching the optimal temperature, the bacterial culture is added to the medium at a ratio of 3%.

13. The method according to any one of the preceding claims, wherein in step (f), the mixture is heated at 70°C for 45 minutes.

14. The method according to any one of the preceding claims, wherein in addition to the fermentation process, at least one of the following techniques is applied: cold pressing, steam distillation, supercritical carbon dioxide extraction, solvent extraction, ultrasonic extraction, and/or microwave extraction.

15. A cosmetic composition, a food composition, a biostimulant composition, or a bioremediation composition comprising at least one fermented plant extract obtained by the method according to any one of preceding claims.
